# EUROPEAN PATENT SPECIFICATION

(11) **EP 0 318 577 B1**
(45) Date of publication and mention of the grant of the patent: **14.09.1994**
(21) Application number: 88906327.7
(22) Date of filing: 01.06.1988
(51) Int. Cl.: F16K 5/10, F16K 35/00, F16K 37/00, A61M 5/14

(54) **FLOW CONTROL DEVICE FOR INTRAVENOUS ADMINISTRATION OF FLUIDS**
FLUSSREGELUNGSANORDNUNG ZUR INTRAVENÖSEN VERABREICHUNG VON FLÜSSIGKEITEN
DISPOSITIF DE REGULATION D'UN DEBIT POUR L'ADMINISTRATION INTRAVEINEUSE DE FLUIDES

(30) Priority: 01.06.1987 US 56129; 09.11.1987 US 118438
(43) Date of publication of application: 07.06.1989
(73) Proprietor: MASTER MEDICAL CORPORATION, Phoenix, AZ 85018 (US)
(72) Inventor: ASLANIAN, Jerry, L., Phoenix, AZ 85018 (US)
(74) Representative: Plucker, Guy
(86) International application number: US8801903
(87) International publication number: WO8809893

(56) References cited:
- WO-A-85/05041
- WO-A-86/00682
- US-A- 545 769
- US-A- 3 276 472
- US-A- 3 305 211
- US-A- 3 540 631
- US-A- 3 698 683
- US-A- 3 785 378
- US-A- 3 788 602
- US-A- 3 807 691
- US-A- 3 980 099
- US-A- 4 256 132

## Description

The present invention relates to a device for regulating and controlling the flow of intravenous (IV) and parenteral fluids. More particularly, the present invention relates to a flow metering device for precisely establishing and maintaining a preselected flow rate during the administration of IV liquids to the patient.

The gravity administration of fluids by IV infusion is a common medical procedure. Representative intravenous fluids include blood, plasma, dextrose and isotonic saline solutions. IV infusions are generally carried out with the container of IV fluid suspended above the patient. Customarily such containers have a seal which is broken by the insertion of a spike and the fluid is delivered to the patient at an administration needle through a drip chamber and flexible tubing connected to the spike. The purpose of the drip chamber is to facilitate the determination of the flow or drip rate through the tubing. The infusion rate is generally regulated by use of an external pinch valve or roller clamp associated with the tubing for the more common gravity type infusions.

Initially when infusions are carried out, the tubing and needle are purged of air by initiating a flow of fluid through the tubing. The needle is then inserted into a venapuncture site at a suitable location such as in the forearm or wrist of the patient and infusion of fluid is initiated. Preferably when the venapuncture site occurs in the lower arm of the patient, the arm should be properly stabilized on a contoured IV arm support. Medical personnel administering the IV will adjust the pinch valve or roller to restrict the IV tubing and the number of drops passing through the drip chamber are counted. The appropriate flow rate is established by trial and error, by progressively restricting or opening the tubing at the pinch valve.

The administration procedure described above requires the attention of medical personnel for a substantial time in the initial establishment of the proper flow rate and in continual monitoring. It is general pratice of medical personnel to periodically check the flow rate by counting the drops of fluid that pass through the drip chamber. Conventional procedures as described not only require substantial time but are often inaccurate. Temperature changes cause expansion and contraction of the IV tubing allowing the flow rate to vary. The tubing may loose "memory" and collapse under continuous squeezing necessitating constant readjustment of the setting. Roller clamps or pinch valves of the general type described have a tendency to slip off the tubing which can pose a threat to the patient.

Independent tests have indicated that conventional pinch valves and roller clamps maintain flow only within about 25% accuracy thereby requiring constant readjustment. Accordingly, it will be appreciated that control of infusion rates with an acceptable degree of accuracy using conventional pinch valves and roller clamps is extremely difficult even with constant attention on the part of attending medical personnel.

Various expedients have been resorted to in an effort to correct the problems set forth above. Flow regulating devices of various types have been developed and can be found in the prior art. U.S. Patent No. 3,785,378 to Stewart shows a valve for the administration of intravenous fluids which has an annular member forming a central passage through which fluid is flowable to an end face having multiple grooves. The inner ends of the multiple grooves communicate with the passage and a flow control member is rotatable to place the flow port successively and selectively in communication with the grooves to vary the flow rate.

Another approach to the problem is found in U.S. Patent No. 3,877,428 to Seagle et al which patent shows an infusion control device for selectively controlling the rate of administration of fluids to a patient. The control device is attachable along the IV tubing and includes a rotatable metering member defining a capillary flow path between the input and output of the control device. A metering plate is axially rotatable with respect to the input and output ports to vary the effective length of the flow path so as to regulate flow between full flow and zero flow conditions.

A somewhat similar approach is shown in U.S. Patent No. 3,880,401 which discloses a flow metering valve having inner and outer component parts which are movable relative one to another at screw threads to effect relative axial movement of a metering valve plug with respect to a metering bore for regulating and terminating flow through the valve flow passage.

Prior U.S. Patent Nos. 4,294,246 and 4,361,147, commonly assigned with the present application, disclose devices which represent substantial improvements over the prior art. A flow passage is provided within a housing and is connectable to a source of IV fluid and to a delivery tube terminating at an administration needle. In the metering apparatus, a metering pin is axially movable within the flow passage relative to a valve seat and defining a flow passageway and variable area flow notch which are positionable relative to the valve seat to regulate flow from a full flow purge position to a flow blocking position. In the preferred embodiment, the positioning of the metering pin is accomplished by a cam engaging a portion of the metering pin which forms a cam follower. The cam is manually adjustable by a dial on the device to accomplish accurate, repeatable and continuous flow adjustment over a full range. The system incorporates the metering apparatus, source of IV fluid, drip chamber and administration means. The apparatus and system works particularly well providing a high degree of accuracy and is competitive even with electronic flow controllers.

From WO 85/05041 there is known a flow regulator which is used for precise adjustement of gravity infusions or transfusions whereby a casing with an inlet port and outlet port contains a cylindrical valve body, the cover of which has a groove of variable cross-section for regulating the flow between the inlet and outlet ports. The groove extends in a plane comprising the outlet port of the casing, perpendicular to the axis of the body at an angle of about 320°. A channel in the form of a groove starting from the largest cross-section of the groove extends parallel to the axis of the body and is linked with a circular groove extending around the periphery of the valve body into which the inlet port leads. Viewed from upstream, the groove is linked with the inlet port via the channel and circular groove and viewed from drownstream and according to the angular position of the body a cross-section of varying size of the groove is linked with the outlet port. This enables a varying number of drops per unit of time to be fed from the casing.

Prior WO 86/00682 patent discloses a metering apparatus and system for controlling the administration of IV fluids having a valve housing with a valving chamber therein. A rotatable valve body member is positioned within the valve chamber and a valve passageway of variable cross-sectional area is provided at either the chamber or on the valve member. The effective area and lenght of the valve passageway interposed between the inlet and outlet to the valve is adjustable to regulate the flow range of rotation only over approximately 180° by moving the valve body relative to the valve member.

The foregoing devices, particularly those described immediately above, provide substantial improvements over conventional pinch valve and roller clamps, nevertheless, are not economically justifiable in some applications or for some medical facilities. Accordingly, there exists a need for a reliable, accurate and repeatable IV flow control device which is simple, effective, accurate and which can be provided to the medical care industry at low cost.

In the present invention, substantially the entire 360° surface of the valving member is used to regulate flow. This permits flow ranges to be spread over a greater circumferential length making the operation of the unit easier and more precise since a small movement will not have as great an effect on the flow rate. Also, because the flow regulation is extended over a full 360°, the appropriate indicia carried on the dial member may be made larger, making them easier to read and providing a greater number of graduations indicating various flow positions.

The present invention concerns an IV metering valve for use in an IV system having a source of fluid connnected to an administration needle via a flexible tubing line, comprising :
(a) a plastic housing 1 defining a generally cylindrical valve chamber, said housing 1 having a inlet 2 and outlet 3 connectable in said tubing line communicating with said valve chamber at spaced-apart locations;
(b) a plastic valving member 7 rotatable within said valve chamber having a generally cylindrical surface defining a flow path consisting of a metering groove 12, extending substantially around said surface and progressively increasing in cross-sectional area and a flow channel 17 axially spaced from said metering groove 12 and being of substantially uniform cross-sectional area and extending circumferentially along the surface of the valving member 7 generally parallel to the metering groove 12, said metering groove 12 and flow channel 17 interconnecting at a bridge 16 wherein said inlet 2 may be selectively registered with a location along said metering groove 12 and said outlet may be selectively registered with a location along said flow channel 17 to regulate flow therethrough from said inlet 2 to said outlet 3, via said flow path and said valving member 7, rotatable to a fast flow purge position with said inlet 2 communicating with said bridge 16.

In particular, said bridge 16 has a cross-sectional area larger than the cross-sectional area of either said metering groove 12 or flow channel 17.

The present invention achieves the above objects and advantages and provides a unique IV control device which can be adjusted to maintain various settings from zero to full flow with accuracy and repeatability.

In accordance with the preferred embodiment, the flow control device of the present invention comprises a housing which has an inlet and outlet and a metering member of a first plastic material. A flow metering member and associated valing member having a variable area flow path is rotatable witin the housing by means of a manually adjustable dial member. The flow metering member selectively places the inlet and outlet in communication via the flow path to regulate the flow from a purge position through a flow adjusting range and to a stop flow position. The inlet and outlet are connectable in the IV tubing line which attaches the source of IV fluid to the administration needle. The valving member is fabricated of a second plastic material which is selected to effect sealing and minimize heat shrinkage. In other aspects of the present invention, a backplate may be selectively attachable to the valve member to provide additional gripping surface for the medical attendant. A security cover may also be associated with the valving member to secure the valving member from tampering or inadvertant adjustment by unauthorized persons.

The above and other objects and advantages of the present invention will become more readily apparent from the following description, claims and drawings in which:
Figures 1 and 2 are exploded views of the present invention;
Figure 3 is an enlarged perspective view of the modified form of the metering drum seen in Figures 1 and 2;
Figures 4 and 5 are cross-sectional views taken along lines 4-4 and 5-5 of Figure 3;
Figures 6 and 7 are cross-sectional views showing different operational conditions of the valve;
Figure 8 is an enlarged perspective view of the rotatable valving member provided with a node;
Figure 9 is a partial transverse sectional view through the inlet fitting showing the valve in an assembled position prior to heating;
Figure 10 is a view similar to Figure 9 after the assembly has been heated;
Figure 11 is a view similar to Figure 10 with the valving member rotated slightly counterclockwise to achieve ultra-low flow rates;
Figure 12 is a sectional view along lines 12-12 of Figure 11;
Figure 13 is an exploded perspective view of the assembly shown in Figure 15.
Figure 14 is a perspective view similar to Figure 15 with the cover in an open position;
Figure 15 is a perspective view of a valve assembly including an optional backplate and cover;
Figure 16 is an exploded view of a valve provided with step means;
Figure 17 is an enlarged cross-sectional view of a portion of a sealing band.

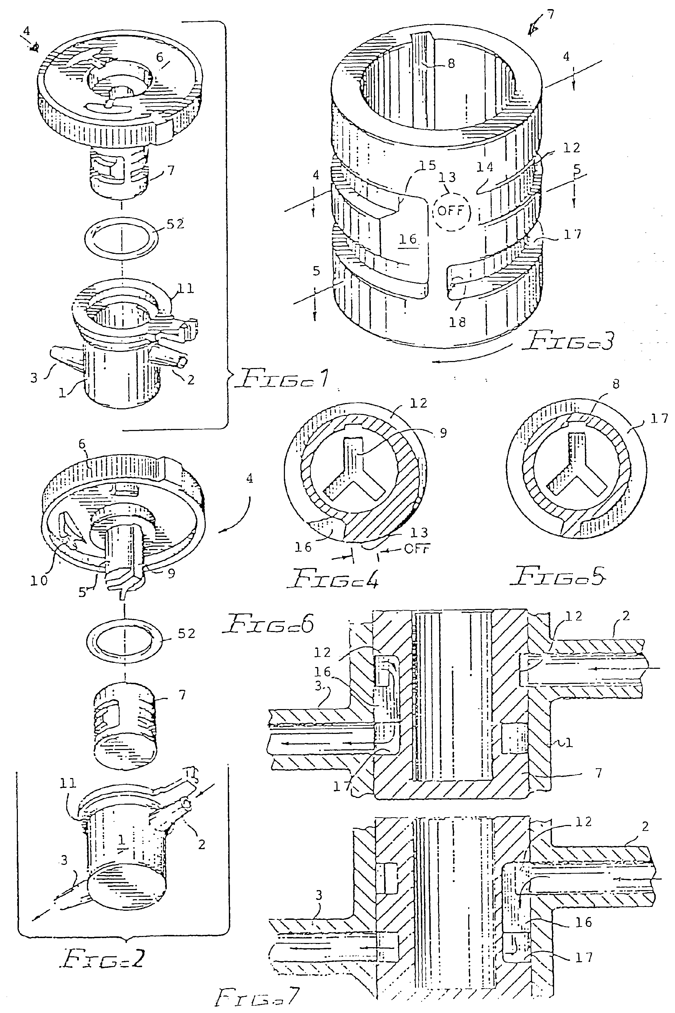

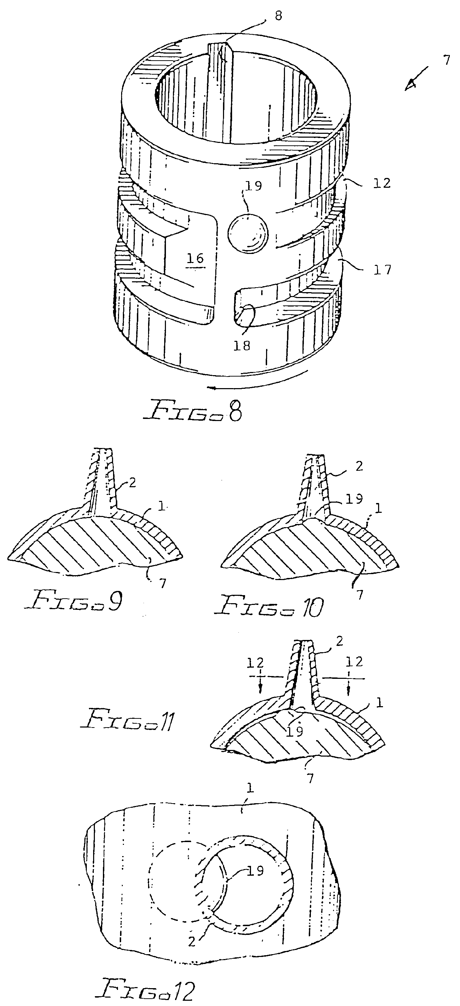

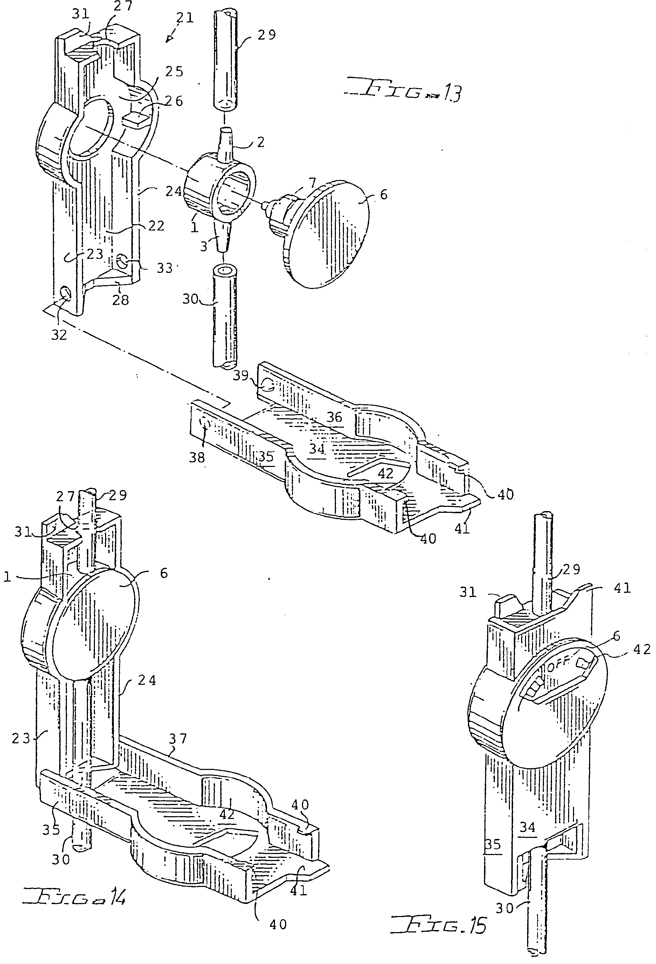

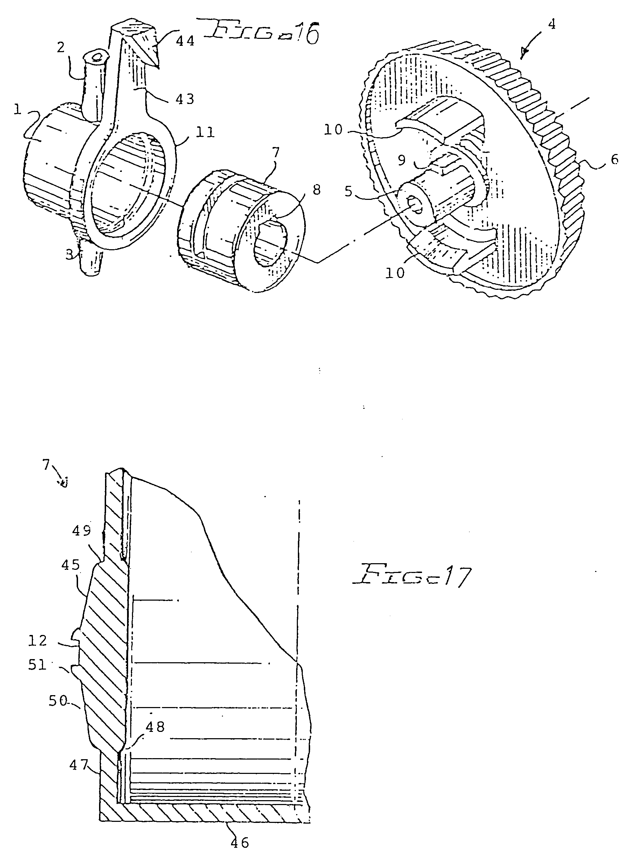

Referring to Figs. 1 to 7, the valve of the present invention is generally having a generally cylindrical valve housing 1 defining a valve chamber which is closed at one end and open at the other end. The inlet 2 and outlet 3 communicate with the valve chamber and are positioned approximately 180° apart with the outlet 3 being axially offset from the inlet 2.

metering member 4 has an axle 5 and an integrally formed dial member 6 which is preferably knurled about its outer periphery to facilitate manual rotation by the user. Cylindrical valving member 7 rotates with axle 5 and is snugly engaged in the valve chamber. The valve housing 1 is preferably a rigid, hard plastic material such as ABS, XT of the like as is the metering member 4 and integral axle 5 and dial member 6. The valving member 7 is preferably of a semi-rigid plastic material such as PVC, LDP, C-Flex®, Santoprene® or other similar material. The valving member 7 may be suitably grooved at recess 8. The axle 5 may be provided with a cooperating projections 9 to be received in recesses 8 for properly aligning these components when they are assembled and to prevent relative rotation. The dial member 6 may include an annular flanged skirt 10 with an inwardly turned lip engageable with a peripheral flange 11 on the valve housing 1 to secure the valve housing 1 in position and prevent separation of the assembled components while permitting rotation of the valving 7 and metering 4 members relative to the valve housing 1. The valve also includes an O-ring 52.

The configuration of the valving member 7 permits the valving member to turn substantially 360° to increase accuracy and to facilitate ease of adjustment although the in-and outlet 2, 3 are spaced only 180° apart. To this end, the outer surface of the valving member 7 is provided with a first circumferential metering groove 12 which begins adjacent land area 13. Metering groove 12 extends circumferentially about the valving member 7 and progressively increases in cross-sectional area from the distal end 14 to area 15 of the metering groove 12 where the metering groove 12 intersects axial bridge section 16. Metering groove 12 may be of various cross-sectional shapes as for example V-shaped, U-shaped of the like and is of varying cross section decreasing progressively from section 15 to section 14. The metering groove 12 communicates axially with flow channel 17 across bridge section 16. Flow channel 17 extends circumferentially from bridge section 16 along outer surface of the valving member 7 generally parallel to the metering groove 12 and terminates at end wall 18 adjacent land area 13. It will be seen that the inlet 2 is axially aligned with the metering groove 12 and the outlet 3 is axially displaced from the inlet 2 to align with the flow channel 17.

Thus, with the valving member 7 in a position with the inlet 2 directly aligned with land area 13, flow through the valve is blocked. As the drum is rotated a short distance in the direction of the arrow as seen in Figure 3, the inlet 2 is moved into registry with metering groove 12, to the position in Figure 6. Accordingly, fluid flows along the metering groove 12 across bridge 16 into the flow channel 17 which is in registry with outlet 3. As the valve is rotated in a clockwise position, the point of registry of inlet 2 and the metering groove 12 is at a location of increasing depth along the metering groove 12 thus permitting an increased flow rate through the metering groove 12, across bridge 16, to flow channel 17 to the outlet 3.

At the point the inlet 2 is in communication with the area 15 of the metering groove where the metering groove intersects bridge section 16, the valve is in the full flow position or purge position as seen in Figure 7. A direct flow path from inlet 2 to outlet 3 is established via flow channel 17. In this position, the system may be purged of air prior to IV administration or a rapid infusion may be administered through the system.

The exterior of the dial member 7 surface may be provided with appropriate indicia indicating various increments of flow rate from closed position to full flow and purge position. Since the metering occures over a full 360° of the dial member 7 surface, more precise setting of the valve is permitted. Further, additional graduations may be provided on the dial surface for the convenience of the user. The flow channel 7 serves as a fluid passage for all flow positions of the valve from initiation of metering through the full range of metering to purge. The valve provides accuracy, repeatability with minimum of moving parts. The components may be conveniently fabricated by plastic molding techniques and the use of plastic materials of dissimilar hardness provides a sealing at the inner face of the valving member 7 and valve chamber.

Another embodiment of the present invention is illustrated in Figures 8 through 12. In this embodiment, the valve member and flow passageways are configured as described with reference to Figures 1 to 7 so that the valving member 7 regulates flow over substantially a 360° range of rotation. The primaraly advantage of the embodiment shown in Figures 8 to 12 is the achievement of a precise, ultra low infusion rate that is highly desirable in intravenous therapy. In the administration of IV fluids, a low flow rate is often necessary, particularly to maintain a KVO (keep vein open) setting. It is not uncommon for patients with long hopitalization periods or other certain physical conditions to develop veins which are collapsed or atrophied. Therefore, medical practice is to maintain the patient connected to an ultra low rate intravenous feeding. The intravenous feeding maintains the patient's vein in an open condition and provides medical personnel a pathway for quick access for administration of medications or drugs. KVO settings are typically in the range of 5 cc. per hour. Such low infusion rates have not been possible with low-cost gravity devices as found in the prior art. Until now, these precise ultra-low rates were only possible with any degree of reliability with computerized volumetric pumps which are expensive and are not available at all medical facilities. Conventional devices such as roller clamps and prior infusion control devices are not capable of achieving with accuracy and repeatability low rates in the range of 5 cc. per hour.

Referring to Figures 8 to 12, the valve of the present invention is generally constructed as has been described with reference to Figures 1 to 7.

With the valving member 7 in the position whereby inlet 2 is directly aligned with land area 13, flow through the valve is blocked. As dial member 6 is rotated a short distance in the direction of the arrow as shown in Fig. 8, inlet 2 is moved out of registry with node 19. Node 19 is located on land area 13 and is thermo-formed and has a shape precisely conforming to the cross-sectional geometry of inlet 2. A similar node 20 exists at the opposite side of the valving member and precisely conforms to outlet 3.

In the "off" position, node 19 is in exact registry with inlet 2 and similarly node 20 is in direct registry with outlet 3. Slight rotation of the dial will move node 19 to the position shown in Figures 11 and 12 which allows a minute volume of fluid to flow from the inlet 2 to flow channel 17. As rotation of the valve continues, nodes 19 and 20 move further out of registry with their associated in- and outlet 2, 3.

As best seen in Figures 8 and 10, the nodes 19 and 20 are in the form of small generally convex protrusions on the surface of the valving member 7. The protrusions, as will be explained hereafter, are formed in situ in a remolding process in the land area of the valving member. The nodes 19, 20 have a peripheral imprint exactly conforming to and, in fact, determined by the precise outlines of the inlet 2 and the outlet 3. In the "off" position, the nodes provide a tight fluid seal. When the nodes are moved slightly out of registry with the associates in- and outlet, as seen in Figs. 11 and 12, limited flow is permitted maintaining an ultra-low KVA rate. Further rotation of the valve will result in the inlet 2 being aligned with the metering groove 12 or bridge 16 and purge and normal flow regulation as described with reference to Figs. 1 to 7 are achieved.

The formation of the nodes in situ is accomplished by applying heat to the assembled valve. When the valve components are assembled, the valving member 7 and housing 1 are engaged in an interference fit in the range of 25 µm to 50 µm. The assembled condition prior to heating is shown in Fig. 9 in which the valving member 7 is tightly positioned in the valve chamber due to the interference fit. After assembly, the valve is subjected to a heating process, typically in the range of 60°C to 82°C for a period of 1/2 to 2 hours. As has been described above, the housing 1 of the valve is formed of a regulatively rigid plastic material and the valving member 7 is formed from a softer material of the class of materials known as thermoplastic elastomers. The prolonged heating of the assembled valve causes the materials, particularly the elastomeric material of the valving member 7, to dimensionally stabilize and, in doing so, exhibit some slight creep or flow. This causes the valving member 7 and housing 1 to closely mate or conform to one another adjusting any small configurational differences that may exist due to dimensional differences that may occur in the fabrication and assembly process. Since the material of the valving member 7 will creep or flow slightly, the plastic material in the area of the in- and outlet 2, 3 will flow slightly, the plastic material in the area of the in- and outlet 2, 3 will flow forming the nodes 19 and 20, as seen in Fig. 10. The peripheral image of in and outlet is transferred to the adjacent surface of the valving member 7 and a slight convexity extends from the periphery. Thus, in the heating process, each valving member 7 becomes perfectly fit or seated within the valve chamber and each valving member 7 is provided with a pair of opposed nodes 19, 20 at the inlet 2 and outlet 3 with nodes have a molded image conforming to the exact contours and irregularities of the associated in-and outlet. This imprint becomes a personal, prominent and individual characteristic of the particular assembly componentry.

Once the heating process is completed, the resulting valve has an individualized sealing or closing barrier to prevent flow of incoming fluid when the valve is in the closed position with the nodes 19, 20 in registry with the associated in- and outlet 2, 3. When the nodes 19, 20 are moved to a position of partial registry, as seen in Figs. 11 and 12 through slight rotation of the valving member 7, fluid is allowed to pass to the metering port in a precise and predictable flow rate.

The in situ formation of the nodes 19, 20 and the remolding of the valving member 7 under heat and pressure provides a highly accurate valve with positive seating. The nodes 19, 20 formed in this manner allow the valve to achieve the ultra KVA flow settings. It is possible to utilize the unique fabrication process with other arrangements such as an arrangement in which the valving member 7 is relatively rigid and the surrounding housing 1 is a softer, elastomeric material with the flow path provided in the surface of the housing 1. In other words, any assembly in which precision fit of the plastic components is required, could utilize the assembly process described above.

The valve is compact and is designed to be manually operated and in some applications its size and weight are an advantage as for example in an extension IV set where the light weight would exert little pull on the needle at the venapuncture site. However, in some other instances, it is desirable that the valve lend itself to one-hand operation. This allows the medical attendant to adjust the valve with one hand while performing other operations with the other hand. To this end, an optional backplate providing additional area for the user to grasp may be provided as can be seen from figures 13, 14 and 15. The backplate of this embodiment is generally designated by the numeral 21 and can be molded as a one-piece unit from a suitable plastic material. The backplate 21 includes a generally planar rear surface 22 and opposite forwardly extending side edges 23 and 24. The side edges are generally parallel and are spaced apart a distance less than the diameter of the housing 1 of the valve. The side edges 23 and 24 both diverge outwardly at an intermediate location having a curvature generally corresponding to the curvature of the circular housing 1 of the valve to form a receptacle 25 for housing 1 of the valve. In this way, the valve can be positioned in the backplate 21 and secured by an interference-fit with the valve housing 1. Projections 26 extend in the receptacle 25 to engage the housing 1 of the valve when inserted into the backplate 21. In the assembled position, the diameter of the dial member 6 exceeds the width of the backplate 21 so that the operator can conveniently grasp the valve with the backplate 21 resting in the palm of the hand and use the thumb and forefinger to grasp the dial member 6 to make the necessary adjustment.

The opposite ends of the backplate 21 are recessed at 27 and 28 to accomodate the incoming and outgoing IV fluid lines 24 and 30. An upwardly extending projection 31 is provided at the upper left hand edge of the backplate 21. Projection 31 serves as part of a release mechanism when the valve is used with a cover as explained below. The lower opposite side edges 23 and 24 are provided with apertures 32 and 33 to accomodate a cover if desired.

The cover may be optionally attached to the backplate 21. The cover may be oppositely attached when it is desired to provide additional security to prevent either intentional or inadvertent adjustment of the setting of the valve as, for example, in the case of juvenile patients. The cover includes a generally flat cover plate 34 having opposite depending sides 35 and 36. A portion of the cover is configured as a generally cylindrical receptacle 37 adapted to receive the dial member 6 of contained valve. The lower ends of opposite sides 35 and 36 depend below the cover plate 34 and are provided with inwardly extending pins 38, 39 which are receivable within apertures 32 and 33 at the opposite side edges 23, 24 of the backplate 21. Since the device is preferably made of a suitable flexible plastic, sides 35 and 36 can be deflected outwardly a sufficient distance to engage the cooperating apertures 32 and 33. Thus, when assembled, the cover is pivotally attached to the backplate 21 and may be positioned in an open position as shown in Figure 14 or pivoted upwardly to a closed position as shown in Figure 15. Locking members 40 at the interior of the sides 35 and 36, engage the edge of the backplate 21 to secure the cover in a closed position. Projection 41 extends upwardly from the upper right hand edge of the cover plate 34 as seen in Figure 14. Thus, to open the cover, projection 31 on the backplate and projection 41 on the cover plate may be easily forced in opposite directions to unlock the cover. A cut-out 42 is provided in the cover plate 34 so that the attendant can visually determine the setting of the valve. Alternatively, the backplate and cover may be molded as one piece and incorporate a connecting "live" hinge of a thin plastic membrane that allows opening and closing of the security cover.

Flange 11 of housing 1 has a vertically extending stop portion 43 which terminates at pointer 44. The rotation of the metering member 4 and associated valving member 7 is limited as annular flanged skirt 10 will engage the stop portion 43. Thus, bi-directional operation is achieved with flow increasing in one direction of operation and decreasing in the other. Appropriate indicia may be placed on the face of dial member 6 which is cooperable with pointer 44 to provide an indication of flow setting.

One particular problem that exists in the use of a valving member 7 of softer material having sealing characteristics, is that in order to maintain sealing an interference fit must exist between the valving member 7 and the inner surface of the housing 1. Often an interference fit imposes a tactile resistance to rotation and interferences with the smooth operation of the valve. Further, because of the close tolerances that must be maintained in parts of this type, any irregularity in the molding process such as non-concentricity of either the housing 1 or the valving member 7 may cause inaccuracy.

To avoid such problems, the valving member 7 of the valve can have a sealing band 45 as best shown in Figure 17 which is an enlarged and slightly exagerated cross-sectional view of a portion of the valving member 7. The valving member 7 as shown has as end wall 46 and cylindrical side wall 47 of generally uniform thickness. However, a circumferential band 45 extends axially in either direction from the metering groove 12 and flow channel 17. The band typically extend approximatively onethird of the overall axial length of the valving member 7. The band 45 projects radially inward at 48 and may also extend radially outward at 49. The outer surface of the band 45 defines an upwardly extending ramp portion 50 terminating at lip 51 which lip defines the opposite axial dimensions of the metering grooves and flow channel 12, 17.

Accordingly, the band 45 of increased radial thickness in the area of the metering groove 12 and flow channel 17 provides increased sealing to prevent leakage. However, because the band 45 does not extend axially the entire lenght of the valving member 7, proper tactile resistance and smoothness of operation is assured. Further, the sealing band 45 will accomodate shrinkage of the part and accomodate any non-concentricity that may occur in the molding process.

Thus, it will be seen from the foregoing that the basic valve as described herein can be economically manufactured and provide the accuracy and repeatibility necessary. In addition, the valve can further be provided with a backplate 21 as an accessory to enhance the operation of the device and to make it compatible with one-hand operation. If security is desired, the optional cover plate 34 can be attached to the backplate 21. In its simplest and most economical form, the valve alone can be used in IV administration. For other applications , the medical attendant can add either the backplate 21 or the backplate 21 and the cover plate 34 as required and dictated by the particular application and procedure.

The unique design and construction of the valving components avoids complex sealing arrangement and are economical to fabricate so the unit can be provided to health care facilities as an inexpensive, disposable item which achieves a repeatable range of flow from KVA to purge. This unit resists tampering and is accurate and simple for the medical attendants to operate giving accurate, repeatable results.

It will be obvious to those skilled in the art to make various changes, alterations and modifications to the invention described herein. To the extend those changes, alterations and modifications do not depart from the scope of the appended claims, they are intended to be encompassed therein.

## Claims

1. An IV metering valve for use in an IV system having a source of fluid connected to an administration needle via a flexible tubing line, comprising :
(a) a plastic housing 1 defining a generally cylindrical valve chamber, said housing (1) having an inlet (2) and outlet (3) connectable in said tubing line communication with said valve chamber at spaced-apart locations;
(b) a plastic valving member (7) rotatable within said valve chamber having a generally cylindrical surface defining a flow path consisting of a metering groove (12) extending substantially around said surface and progressively increasing in cross-sectional area and a flow channel (17) axially spaced from said metering groove (12) and being of substantially uniform cross-sectional area and extending circumferentially along the surface of the valving member (7) generally parallel to the metering groove (12), said metering groove (12) and flow channel (17) interconnecting at a bridge (16), said valving member (7) being rotatable to a fast flow purge position with said inlet (2) communicating with said bridge (16),
characterized in that :
(c) said inlet (2) may be selectively registered with a location along said metering groove (12) and said outlet 3 may be selectively registered with a location along said flow channel (17) to regulate flow therethrough from said inlet (2) to said outlet (3) via said flow path, and
(d) said bridge (16) has a cross-sectional area larger than the cross-sectional area of either said metering groove (12) or flow channel (17).

2. A valve according to claim 1, characterized in that said housing (1) is made of a first plastic material and said valving member (7) is made of a second relatively softer plastic material to provide a sealing and wiping action.

3. A valve according to any one of the preceding claims, characterized in that said flow channel (17) terminates at an end wall (18) adjacent to a land area (13) longitudinally aligned with said metering groove (12) and registerable with said inlet (2) in the off position.

4. A valve according to any one of the preceding claims, characterized in that said valving member (7) defines an axially extending recess (8).

5. A valve according to any one of the preceding claims, characterized in that it comprises a cylindrical metering member (4) with a projection (9) provided on the outer cylindrical surface of a cylindrical axle (5) of said metering member to accomodate alignment of the valving member (7) with said metering member (4) and to resist relative rotation between these components.

6. A valve according to any one of the preceding claims, characterized in that said metering member (4) also includes a dial member (6) for selectively moving said metering member (4) relative to said housing (1) to interpose a selected portion of said flow path to vary the flow rate and for providing an indication of the flow rate.

7. A valve according to any one of the preceding claims, characterized in that said cylindrical axle (5) has a generally Y-shaped projection (9) at its outer end which facilitates proper alignment of the axle (5) with the cylindrical valving member (7), having a generally cylindrical body with an open upper end and a closed bottom end, said closed bottom end having a recess (8) therein conforming to the generally Y-configuration of the projection (9) on the end of the axle (5) in order to ensure proper alignment of the two components and to prevent relative rotation between these members when the valving member (7) is placed over the axle (5) and the components are forced together.

8. A valve according to any of the preceding claims, wherein said housing (1) is provided with a stop portion (43) cooperable with said metering member (4) to limit movement thereof.

9. A valve according to any one of the preceding claims, characterized in that it comprises a valve guard having a backplate (21) defining a receptacle (25) for receiving the housing (1) of said valve in a position with the dial member (6) accessible, said backplate (21) extending longitudinally opposite said receptacle (25) providing a gripping surface to allow the valve to be manually adjustable at said dial member (6).

10. A valve of claim 9, characterized in that it comprises a cover plate (34) detachably securable to said backplate (21) at pivotal means (27,28,38,39) whereby the cover plate (34) is positionable in an open position exposing said dial member (6) to permit adjustment of said valve and having a closed position covering said valve to prevent inadvertent adjustment of the dial member (6).

11. A valve of claim 10, wherein said cover plate (34) is provided with a cut-out portion (42) therein exposing at least a part of the dial member (6) of said valve in said closed position.

12. A valve according to any one of claims 9, 10 and 11, characterized in that it further includes cooperable locking members (40) on said cover plate (34) and said backplate (21) for securing said backplate (21) and cover plate (34) in said closed position.

13. A valve according any one of the preceding claims, characterized in that said valving member (7) has a node (25) on the said land area (13) conforming to the configuration of the inlet (2) and being in registry therewith in the off position and whereby predetermined rotation of said valving member (7) will displace said node (25) from registry with said inlet (2) establishing an ultra-low flow condition.

14. A valve according any one of the claims 1 to 12, characterized in that a node (20) is provided on said valving member (7) conforming to the configuration of the outlet (3) and being in registry therewith in the off position.

15. A valve according to any of the preceding claims, wherein said first plastic material is selected from the group consisting of ABS, XT and said second plastic material is selected from the group consisting of LDP or PVC.

16. A valve according to any one of the preceding claims, wherein said dial member (6) is provided with a flanged skirt (10) which engages a portion of said valve housing (1).

17. A valve according to any of the preceding claims, characterized in that said valving member (4) is configured having a sealing band (45) of increased radial thickness in the area of the flow channel (17) and metering groove (12), said band (45) extending axially in either direction from the metering groove (12) and flow channel (17) and extending approximately one-third of the overall axial length of the metering member (7), the outer surface of the band defining an upwardly extending ramp portion (50) terminating at lip (51) which lip defines the opposite axial dimensions of the metering groove (12) and flow channel (17), said band 45 providing increased sealing to prevent leakage, assuring proper tactile resistance and smoothness of operation and accomodating shrinkage of the part and any non-concentricity that may occur in the molding process.

## Patentansprüche

1. IV-Dosierventil zur Verwendung in einem IV-System mit einer über eine flexible Schlauchleitung mit einer Verabreichungsnadel verbundenen Fluidquelle, bestehend aus:
a) einem eine im allgemeinen zylindrische Ventilkammer begrenzenden Kunststoffgehäuse (1), wobei das Gehäuse (1) einen Einlaß (2) und einen Auslaß (3) aufweist, die mit der Schlauchleitung verbindbar sind und mit der Ventilkammer an mit Abstand voneinander angeordneten Stellen in Verbindung stehen;
b) einem in der Ventilkammer drehbaren Kunststoffventilglied (7) mit einer im allgemeinen zylindrischen Oberfläche, die einen Strömungsweg bildet, bestehend aus einer im wesentlichen rund um die Oberfläche verlaufenden und fortschreitend in der Querschnittsfläche größer werdenden Dosiernut (12) und einem in axialer Richtung von der Dosiernut (12) beabstandeten, eine im wesentlichen gleichförmige Querschnittsfläche aufweisenden und in Umfangsrichtung entlang der Oberfläche des Ventilgliedes (7) im allgemeinen parallel zur Dosiernut verlaufenden Strömungskanal (17), wobei die Dosiernut (12) und der Strömungskanal (17) an einer Brücke (16) miteinander verbunden sind und das Ventilglied (7) in eine Spülstellung mit schneller Strömung drehbar ist, in der der Einlaß (2) mit der Brücke (16) in Verbindung steht,
dadurch gekennzeichnet, daß
c) der Einlaß (2) nach Wahl mit einer Stelle entlang der Dosiernut (12) und der Auslaß (3) nach Wahl mit einer Stelle entlang dem Strömungskanal (17) in Deckung gebracht werden kann, um die Strömung durch diese vom Einlaß (2) zum Auslaß (3) über den Strömungsweg zu regulieren, und
d) die Brücke (16) eine Querschnittsfläche hat, die größer ist als die Querschnittsfläche der Dosiernut (12) oder des Strömungskanals (17).

2. Ventil nach Anspruch 1, dadurch gekennzeichnet, daß das Gehäuse (1) aus einem ersten Kunststoffmaterial und das Ventilglied (7) aus einem zweiten im Verhältnis weicheren Kunststoffmaterial hergestellt ist, um eine Abdichtungs- und Wischwirkung herbeizuführen.

3. Ventil nach einem der vorhergehenden Ansprüche, dadurch gekennzeichnet, daß der Strömungskanal (17) an einer Endwand (18) angrenzend an einen Stegbereich (13) endet, der in Längsrichtung mit der Dosiernut (12) ausgerichtet und mit dem Einlaß (2) in der Aus-Stellung zur Deckung bringbar ist.

4. Ventil nach einem der vorhergehenden Ansprüche, dadurch gekennzeichnet, daß das Ventilglied (7) eine axial verlaufende Ausnehmung (8) bildet.

5. Ventil nach einem der vorhergehenden Ansprüche, dadurch gekennzeichnet, daß es ein zylindrisches Dosierglied (4) mit einem Vorsprung (9) auf der äußeren zylindrischen Oberfläche einer zylindrischen Achse (5) des Dosierglieds umfaßt, um eine Ausrichtung des Ventilglieds (7) mit dem Dosierglied (4) vorzunehmen und eine Relativdrehung zwischen diesen Bauteilen zu verhindern.

6. Ventil nach einem der vorhergehenden Ansprüche, dadurch gekennzeichnet, daß das Dosierglied (4) ferner ein Einstellglied (6) zum wahlweisen Bewegen des Dosierglieds (4) in bezug auf das Gehäuse (1) aufweist, um einen ausgewählten Bereich des Strömungsweges zum Verändern des Strömungsdurchsatzes einzustellen und eine Anzeige des Strömungsdurchsatzes herbeizuführen.

7. Ventil nach einem der vorhergehenden Ansprüche, dadurch gekennzeichnet, daß die zylindrische Achse (5) einen Vorsprung (9) von Y-förmiger Grundgestalt an ihrem äußeren Ende aufweist, der die ordnungsgemäße Ausrichtung der Achse (5) mit dem zylindrischen Ventilglied (7) erleichtert, das ein Gehäuse von zylindrischer Grundform mit einem offenen oberen Ende und einem geschlossenen unteren Ende besitzt, wobei das geschlossene untere Ende eine Ausnehmung (8) besitzt, die der Y-Grundgestalt des Vorsprungs (9) am Ende der Achse (5) entspricht, um die zutreffende Ausrichtung der beiden Bauteile zu gewährleisten und eine Relativdrehung zwischen diesen Teilen zu verhindern, wenn das Ventilglied (7) auf die Achse (5) aufgesetzt wird und die Bauteile zusammengedrückt werden.

8. Ventil nach einem der vorhergehenden Ansprüche, bei dem das Gehäuse (1) mit einem Anschlagbereich (43) versehen ist, der mit dem Dosierglied (4) zur Begrenzung dessen Bewegung zusammenwirken kann.

9. Ventil nach einem der vorhergehenden Ansprüche, dadurch gekennzeichnet, daß es einen Ventilschutz mit einer Rückenplatte (21) umfaßt, die ein Behältnis (25) zur Aufnahme des Gehäuses (1) des Ventils in einer Stellung mit zugänglichem Einstellglied (6) bildet, wobei die Rückenplatte (21) in Längsrichtung gegenüber dem Behältnis (25) verläuft und eine Grifffläche bietet, damit das Ventil von Hand am Einstellglied (6) einstellbar sein kann.

10. Ventil nach Anspruch 9, dadurch gekennzeichnet, daß es eine Abdeckplatte (34) umfaßt, die an der Rückenplatte (21) an Schwenkmitteln (27,28,38,39) lösbar festlegbar ist, wodurch die Abdeckplatte (34) in eine das Einstellglied (6) zur Ermöglichung einer Einstellung des Ventils freilegende Offenstellung bringbar ist und eine geschlossene Stellung aufweist, in der das Ventil abgedeckt ist, um eine unabsichtliche Verstellung des Einstellglieds (6) zu verhindern.

11. Ventil nach Anspruch 10, bei dem die Abdeckplatte (34) mit einem Ausschnittsbereich (42) versehen ist, der zumindest einen Teil des Einstellglieds (6) des Ventils in der geschlossenen Stellung freilegt.

12. Ventil nach einem der Ansprüche 9, 10 und 11, dadurch gekennzeichnet, daß es des weiteren zusammenwirkende Verriegelungsglieder (40) an der Abdeckplatte (34) und der Rückenplatte (21) zur Festlegung der Rückenplatte (21) und der Abdeckplatte (34) in der geschlossenen Stellung aufweist.

13. Ventil nach einem der vorhergehenden Ansprüche, dadurch gekennzeichnet, daß das Ventilglied (7) einen Ansatz (19) auf der Stegfläche (13) besitzt, der der Konfiguration des Einlasses (2) entspricht und sich mit diesem in der Aus-Stellung in Deckung befindet, wobei eine vorbestimmte Drehung des Ventilglieds (7) den Ansatz (19) aus seiner Deckung mit dem Einlaß (2) verlagert und einen ultralangsamen Strömungszustand herstellt.

14. Ventil nach einem der Ansprüche 1 bis 12, dadurch gekennzeichnet, daß auf dem Ventilglied (7) ein Ansatz (20) vorgesehen ist, der der Konfiguration des Auslasses (3) entspricht und sich mit diesem in der Aus-Stellung in Deckung befindet.

15. Ventil nach einem der vorhergehenden Ansprüche, bei dem das erste Kunststoffmaterial aus der aus ABS, XT bestehenden Gruppe und das zweite Kunststoffmaterial aus der aus LDP oder PVC bestehenden Gruppe ausgewählt ist.

16. Ventil nach einem der vorhergehenden Ansprüche, bei dem das Einstellglied (6) mit einem Flanschrand (10) versehen ist, der mit einem Bereich des Ventilgehäuses (1) in Eingriff steht.

17. Ventil nach einem der vorhergehenden Ansprüche, dadurch gekennzeichnet, daß das Ventilglied (4) so gestaltet ist, daß es einen Dichtungsbund (45) von vergrößerter radialer Dicke im Bereich des Strömungskanals (17) und der Dosiernut (12) aufweist, der Bund (45) axial in beiden Richtungen von der Dosiernut (12) und dem Strömungskanal (17) verläuft und sich über etwa 1/3 der gesamten axialen Länge des Dosierglieds (7) erstreckt und die äußere Oberfläche des Bundes einen aufwärtsgerichteten, an einer Lippe (51) endenden Rampenbereich (50) bildet, welche Lippe die gegenüberliegenden axialen Abmessungen der Dosiernut (12) und des Strömungskanals (17) definiert, wobei der Bund (45) eine verstärkte Abdichtung bereitstellt, um eine Undichtigkeit zu verhindern, einen angemessenen fühlbaren Widerstand und einen glatten Betrieb gewährleistet sowie einen Schrumpf des Teils und eine Nicht-Konzentrizität, die im Formgebungsprozeß eintreten kann, auffängt.

## Revendications

1. Valve à débit réglable à utiliser dans un système intraveineux comportant une source de fluide raccordée à une aiguille d'administration par l'intermédiaire d'un tuyau flexible, comprenant :
(a) un corps en matière plastique (1) délimitant une chambre de valve sensiblement cylindrique, le corps (1) comportant une entrée (2) et une sortie (3) pouvant être branchées dans le tuyau flexible communiquant avec la chambre de valve en des endroits espacés;
(b) une noix de valve en matière plastique (7) pouvant tourner dans la chambre de valve, comportant une surface dans l'ensemble cylindrique définissant un trajet d'écoulement constitué d'une rainure de réglage de débit (12) qui s'étend en substance autour de la surface et dont la section en coupe transversale va en augmentant progressivement et un canal d'écoulement (17) espacé axialement de la rainure (12), d'une section en coupe transversale en substance uniforme et s'étendant circonférentiellement le long de la surface de la noix de valve (7), en substance parallèlement à la rainure de réglage de débit (12), la rainure de réglage de débit (12) et le canal d'écoulement (17) communiquant l'un avec l'autre au niveau d'un pont (16), la noix de valve pouvant tourner vers une position de purge à écoulement rapide dans laquelle l'entrée (2) est en communication avec le pont (16),
caractérisé en ce que :
(c) l'entrée (2) peut être amenée sélectivement en coïncidence avec un endroit le long de la rainure de réglage de débit (12) et la sortie (3) peut être amenée sélectivement en coïncidence avec un endroit le long du canal (17) pour régler le débit qui le traverse depuis l'entrée (2) vers la sortie (3) via le trajet d'écoulement, et
(d) le pont (16) présente une section en coupe transversale supérieure à la section en coupe transversale de la rainure de réglage de débit (12) ou du canal d'écoulement (17).

2. Valve suivant la revendication 1, caractérisée en ce que le corps (1) est fait d'une première matière plastique et la noix de valve (7) est faite d'une seconde matière plastique relativement plus souple afin d'assurer un effet d'étanchéité et de frottement.

3. Valve suivant l'une quelconque des revendications précédentes, caractérisée en ce que le canal d'écoulement (17) se termine à une paroi d'extrémité (18) adjacente à une zone non rainurée (13) longitudinalement en ligne avec la rainure de réglage de débit (12) et pouvant être amenée en coïncidence avec l'entrée (2) dans la position de fermeture.

4. Valve suivant l'une quelconque des revendications précédentes, caractérisée en ce que la noix de valve (7) définit un évidement (8) qui s'étend dans le sens axial.

5. Valve suivant l'une quelconque des revendications précédentes, caractérisée en ce qu'elle comprend un organe de réglage cylindrique (4) pourvu d'une saillie (9) prévue sur la surface cylindrique externe d'un axe cylindrique (5) dudit organe de réglage pour assurer l'alignement de la noix de valve (7) avec l'organe de réglage (4) et pour s'opposer à une rotation de ces éléments l'un par rapport à l'autre.

6. Valve suivant l'une quelconque des revendications précédentes, caractérisée en ce que l'organe de réglage (4) comprend également un cadran (6) afin de déplacer sélectivement l'organe de réglage (4) par rapport au corps (1) pour interposer une partie sélectionnée du trajet d'écoulement en vue de modifier le débit et de fournir une indication du débit.

7. Valve suivant l'une quelconque des revendications précédentes, caractérisée en ce que l'axe cylindrique (5) comporte une saillie en substance en Y (9), à son extrémité externe, qui facilite l'alignement adéquat de l'axe (5) avec la noix de valve cylindrique (7) qui comporte un corps dans l'ensemble cylindrique avec une extrémité supérieure ouverte et une extrémité inférieure fermée, l'extrémité inférieure fermée présentant un évidement (8) qui épouse d'une manière générale la forme en Y de la saillie (9) prévue sur l'extrémité de l'axe (5) afin d'assurer un alignement approprié des deux éléments et d'empêcher tout mouvement de rotation de ces éléments l'un par rapport à l'autre lorsque la noix de valve (7) est placée sur l'axe (5) et que les éléments sont emboîtés de force l'un dans l'autre.

8. Valve suivant l'une quelconque des revendications précédentes, dans laquelle le corps (1) est pourvu d'une partie d'arrêt (43) pouvant coopérer avec l'organe de réglage (4) pour limiter son déplacement.

9. Valve suivant l'une quelconque des revendications précédentes, caractérisée en ce qu'elle comprend un habillage de valve comportant une plaque dorsale (21) délimitant un logement (25) destiné à recevoir le corps (1) de la valve dans une position dans laquelle le cadran (6) est accessible, la plaque dorsale (21) qui s'étend longitudinalement en face du logement (25) offrant une surface de prise permettant de régler la valve à la main au moyen du cadran (6).

10. Valve suivant la revendication 9, caractérisée en ce qu'elle comprend une plaque frontale (34) pouvant être fixée de façon détachable à la plaque dorsale (21) au niveau de moyens de pivotement (27, 28, 38, 39), la plaque frontale (34) pouvant être placée dans une position ouverte exposant le cadran (6) afin de permettre le réglage de la valve et comportant une position fermée couvrant la valve pour empêcher un réglage fortuit du cadran (6).

11. Valve suivant la revendication 10, dans laquelle la plaque frontale (34) est pourvue d'une découpe (42) exposant au moins une partie du cadran (6) de la valve en position fermée.

12. Valve suivant l'une quelconque des revendications 9, 10 et 11, caractérisée en ce qu'elle comprend, en outre, des organes de verrouillage coopérants (40) sur la plaque frontale (34) et la plaque dorsale (21) pour fixer la plaque dorsale (21) et la plaque frontale (34) dans la position de fermeture.

13. Valve suivant l'une quelconque des revendications précédentes, caractérisée en ce que la noix de valve (7) présente une bossette (19) sur la zone non rainurée (13) épousant la forme de l'entrée (2) et placée en coïncidence avec celle-ci dans la position de fermeture, une rotation prédéterminée de la noix de valve (7) écartant la bossette (19) de sa position de coïncidence avec l'entrée (2) et établissant des conditions d'écoulement ultrafaible.

14. Valve suivant l'une quelconque des revendications 1 à 12, caractérisée en ce qu'une bossette (20) est prévue sur la noix de valve (7), épouse la configuration de la sortie (3) et coïncide avec celle-ci dans la position de fermeture.

15. Valve suivant l'une quelconque des revendications précédentes, dans laquelle la première matière plastique est choisie dans le groupe comprenant l'ABS et le XT et la seconde matière plastique est choisie dans le groupe comprenant le LDP et le PVC.

16. Valve suivant l'une quelconque des revendications précédentes, dans laquelle le cadran (6) est pourvu de languettes (10) qui sont en prise avec une partie du corps de valve (1).

17. Valve suivant l'une quelconque des revendications précédentes, caractérisée en ce que la noix de valve (7) est agencée avec une bande d'étanchéité (45) d'épaisseur radiale accrue dans la zone du canal d'écoulement (17) et de la rainure de réglage de débit (12), la bande (45) s'étendant axialement dans les deux sens à partir de la rainure de réglage de débit (12) et du canal d'écoulement (17) et s'étendant approximativement sur un tiers de la longueur axiale totale de la noix de valve (7), la surface externe de la bande définissant une partie formant rampe s'étendant vers le haut (50) qui se termine à une lèvre (51) définissant les dimensions axiales opposées de la rainure de réglage de débit (12) et du canal d'écoulement (17), la bande (45) offrant une étanchéité accrue pour empêcher les fuites, assurant une résistance tactile et une douceur de fonctionnement adéquates et compensant le retrait de la pièce ainsi que tout défaut de concentricité pouvant survenir dans le processus de moulage.
